# EUROPÄISCHE PATENTSCHRIFT

(11) **EP 2 448 820 B1**
(45) Veröffentlichungstag und Bekanntmachung des Hinweises auf die Patenterteilung: **16.01.2013**
(21) Anmeldenummer: 10724330.5
(22) Anmeldetag: 03.05.2010
(51) Int. Cl.: B65B 3/00, B65B 7/28, B65B 7/16

(54) **VORRICHTUNG ZUM FÜLLEN UND VERSCHLIESSEN VON PHARMAZEUTISCHEN BEHÄLTNISSEN**
DEVICE FOR FILLING AND SEALING PHARMACEUTICAL CONTAINERS
DISPOSITIF POUR REMPLIR ET FERMER DES CONTENANTS PHARMACEUTIQUES

(30) Priorität: 03.07.2009 DE 102009027452
(43) Veröffentlichungstag der Anmeldung: 09.05.2012
(73) Patentinhaber: Robert Bosch GmbH, 70442 Stuttgart (DE)
(72) Erfinder: KRAUSS, Ulrich, 74532 Ilshofen (DE); HUMPFER, Steffen, 74589 Satteldorf (DE); ULLHERR, Klaus, 74564 Crailsheim (DE); MAYER, Werner, 74564 Crailsheim (DE)
(86) Internationale Anmeldenummer: PCT/EP2010/055955
(87) Internationale Veröffentlichungsnummer: WO 2011/000606

(56) Entgegenhaltungen:
- DE-A1- 4 419 475
- DE-A1- 10 330 700
- DE-A1-102005 026 986
- DE-A1-102007 016 249
- DE-A1-102008 001 287

## Beschreibung

### Stand der Technik

Die Erfindung betrifft eine Vorrichtung zum Füllen und Verschließen von pharmazeutischen Behältnissen nach dem Oberbegriff des Anspruchs 1.

Eine derartige Vorrichtung ist bereits allgemein bekannt und wird in der pharmazeutischen Industrie zum Füllen und Verschließen von Spritzenkörpern eingesetzt. Hierbei sind die Spritzenkörper in einem Trägerelement (einem sogenannten Nest) angeordnet, wobei das Trägerelement wiederum in einem Aufnahmebehälter (einem sogenannten Tub) angeordnet ist. Eine derartige Anordnung der Spritzenkörper hat den Vorteil, dass die Spritzenkörper bereits beim Glas- bzw. Kunststoffhersteller vorsterilisiert werden können, so dass sie beim Pharmazeuten lediglich noch abgefüllt und verschlossen werden müssen.

Neben den erwähnten Spritzenkörpern sind des Weiteren pharmazeutische Behältnisse in Form von sogenannten Vials sowie Zylinderampullen bekannt, die im Gegensatz zu Spritzenkörpern mit einem zusätzlichen Verschlusselement in Form einer Bördelkappe versehen sind. Eine Verarbeitung bzw. Handhabung aller drei erwähnten Behältnisse auf ein und derselben Vorrichtung ist mit der vorbekannten Vorrichtung zum Füllen und Verschließen von pharmazeutischen Behältnissen in den oben genannten Trägerelementen nicht möglich.

### Offenbarung der Erfindung

Ausgehend von dem dargestellten Stand der Technik liegt der Erfindung die Aufgabe zugrunde, eine Vorrichtung zum Füllen und Verschließen von pharmazeutischen Behältnissen nach dem Oberbegriff des Anspruchs 1 derart weiterzubilden, dass diese auch für die Handhabung bzw. für das Füllen und Verschließen von mit Bördelkappen zu versehenden pharmazeutischen Behältnissen, insbesondere für Vials und Zylinderampullen geeignet ist. Diese Aufgabe wird bei einer Vorrichtung zum Füllen und Verschließen von pharmazeutischen Behältnissen mit den Merkmalen des Anspruchs 1 gelöst. Der Erfindung liegt dabei die Idee zugrunde, der zweiten Handhabungseinrichtung eine Bördeleinrichtung zuzuordnen, welche in Abhängigkeit von dem jeweils zu verarbeitenden Behältnis mittels der zweiten Handhabungseinrichtung angesteuert werden kann.

Vorteilhafte Weiterbildungen der erfindungsgemäßen Vorrichtung zum Füllen und Verschließen von pharmazeutischen Behältnissen sind in den Unteransprüchen angegeben. In den Rahmen der Erfindung fallen sämtliche Kombinationen aus zumindest zwei von in der Beschreibung, den Ansprüchen und/oder den Figuren offenbarten Merkmalen.

Um eine flexible Gestaltung der Taktzeiten sowie der Ausgestaltung der Bördeleinrichtung zu ermöglichen, ist es in einer vorteilhaften Weiterbildung vorgesehen, dass der Bördeleinrichtung eine Zufördereinrichtung mit Aufnahmeelementen für die Behältnisse vorgeschaltet ist und, dass die Aufnahmeelemente der Zufördereinrichtung einen Übernahmebereich für die Behältnisse aus dem Trägerelement und einen Übergabebereich der Behältnisse an das Trägerelement aufweist, in der die Aufnahmeelemente während der Übergabe bzw. Übernahme der Behältnisse mittels der zweiten Handhabungseinheit zusammen mit der zweiten Handhabungseinheit aneinander vorbeibewegt werden. Dadurch wird es beispielsweise möglich, dass die Übernahme bzw. Übergabe der Behältnisse während einer Bewegung eines Trägerelements erfolgt, während sich ein anderes Trägerelement gleichzeitig in der Bördeleinrichtung befindet und dort stillsteht.

Insbesondere ist es dabei vorteilhaft, wenn die zweite Handhabungseinheit als Handhabungsroboter ausgebildet ist, dessen mit den Behältnissen in Wirkverbindung angeordneter Greifarm bezüglich seines Bewegungswegs horizontal und vertikal frei programmierbar ist. Dadurch lässt sich die erfindungsgemäße Vorrichtung nicht nur grundsätzlich bezüglich der Art der zu verarbeitenden Behältnisse anpassen, sondern es ist darüber hinaus auch möglich, bei ein und derselben Behältnisart Anpassungen an unterschiedliche Formate der Behältnisse auf einfache Art und Weise vornehmen zu können.

Damit sichergestellt ist, dass die Wahrscheinlichkeit eines Verlustes einer Bördelkappe möglichst gering ist sowie die Bördelkappen auf nur einem möglichst geringen Weg innerhalb der Vorrichtung gefördert werden, ist es darüber hinaus vorteilhaft, dass der Bördeleinrichtung unmittelbar eine Bördelkappenübergabeeinrichtung vorgeschaltet ist, in der die Behältnisse jeweils eine Bördelkappe übernehmen.

In einer alternativen Ausführungsform, die bezüglich der Störung des Laminarflows innerhalb des Gehäuses besonders vorteilhaft ist, ist es jedoch vorgesehen, dass der Greifarm vor der Entnahme der Behältnisse auf dem Trägerelement an einer Bördelkappenbevorratungseinrichtung vorbeigeführt wird, in der der Greifarm Bördelkappen entnimmt und, dass der Greifarm vor der Entnahme der Behältnisse aus dem Trägerelement auf den Kopfbereich jedes Behältnisses eine Bördelkappe aufsetzt. Da der Greifarm bei der Entnahme der Behältnisse aus dem Trägerelement in jedem Fall in Überdeckung mit den Behältnissen gebracht werden muss, findet durch die gleichzeitige Übergabe der Kappen an die Behältnisse keine Verschlechterung des Laminarflows statt.

Besonders vorteilhaft ist es hierbei, wenn der Greifarm beim Aufsetzen der Bördelkappe auf das Behältnis die Bördelkappe durch Anbördeln an gegenüberliegenden Seiten die Bördelkappe in Wirkverbindung mit dem Behältnis bringt, so dass die Bördelkappe auf dem Behältnis gesichert ist. Dadurch wird vermieden, dass nach der Entnahme der Behältnisse aus dem Trägerelementes zu einem Verlust der Bördelkappe zwischen der Entnahme der Behältnisse und der Bördeleinrichtung kommt.

Ein pharmazeutisch sicherer Betrieb, der darüber hinaus keinerlei baulichen Veränderungen an bestehenden Fabrikgebäuden notwendig macht, wird erzielt, wenn die Vorrichtung mit Ausnahme einer Einführschleuse und einer Ausführschleuse in einem geschlossenen Gehäuse aufgenommen ist und, dass eine Gebläseeinrichtung vorgesehen ist, die innerhalb des Gehäuses einen vom Deckenbereich in den Bodenbereich gerichteten Laminarflow erzeugt.

Um die Anpassung an verschiedenste Formate der Behältnisse sowie grundsätzlich auch an unterschiedliche Behältnisse zu ermöglichen, ist es darüber hinaus besonders vorteilhaft, dass zumindest die Füll- und Verschließeinrichtung, die Bördeleinrichtung und die der Bördeleinrichtung zugeordneten Einrichtungen an standardisierten Aufnahmeelementen austauschbar befestigt sind.

Um einen möglichst kompakten Aufbau der Vorrichtung zu ermöglichen sowie eine gute Übersichtlichkeit und einen guten Laminarflow zu erreichen, ist es darüber hinaus vorteilhaft, dass die Fördereinrichtung für den Aufnahmebehälter geradlinig ausgebildet ist und sich zwischen der Einführschleuse und der Ausführschleuse im Gehäuse erstreckt und, dass zumindest die Handhabungseinrichtungen, die Füll- und Verschließeinrichtung und die Bördeleinrichtung sowie die der Bördeleinrichtung zugeordneten Einrichtungen seitlich neben der Fördereinrichtung auf einer gemeinsamen Seite innerhalb des Gehäuses angeordnet sind.

Mittels einer Ausbildung, in der die erste Handhabungseinrichtung als Handhabungsroboter ausgebildet ist, dessen mit den Behältnissen in Wirkverbindung angeordneter Greifarm bezüglich seines Bewegungswegs horizontal und vertikal frei programmierbar ist und, dass im Bewegungsweg des Greifarms wenigstens eine Wiegeeinrichtung für die Behältnisse angeordnet ist, wird der Vorteil erzielt, dass eine Kontrolle der Behältnisse bezüglich der Füllmenge möglich wird und die Handhabungseinrichtung einfach an unterschiedliche Behältnisse angepasst werden kann.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:

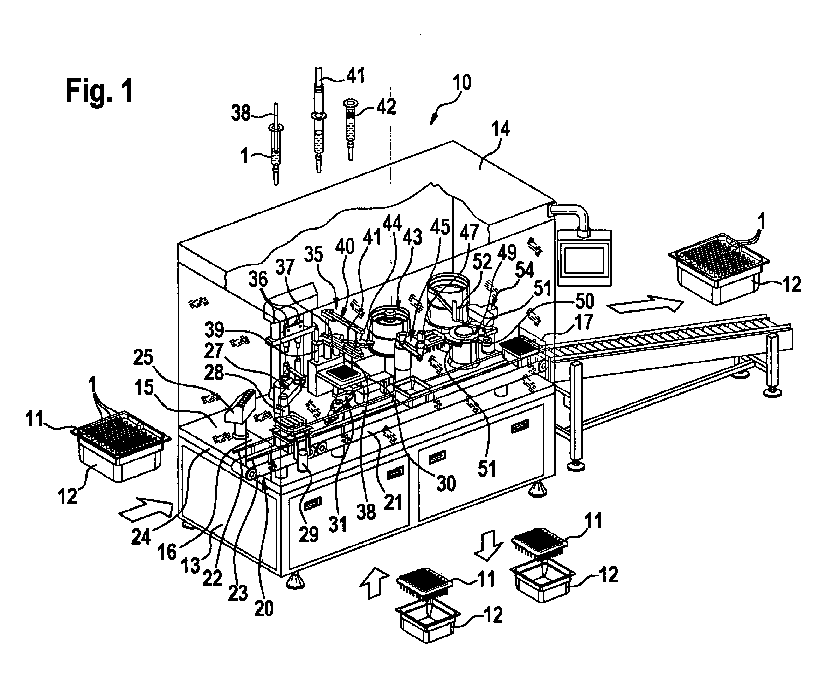

- Fig. 1: eine erfindungsgemäße Vorrichtung zum Füllen und Verschließen von pharmazeutischen Behältnissen bei der Verarbeitung von Spritzenkörpern in einer teilweise aufgeschnittenen perspektivischen Darstellung,

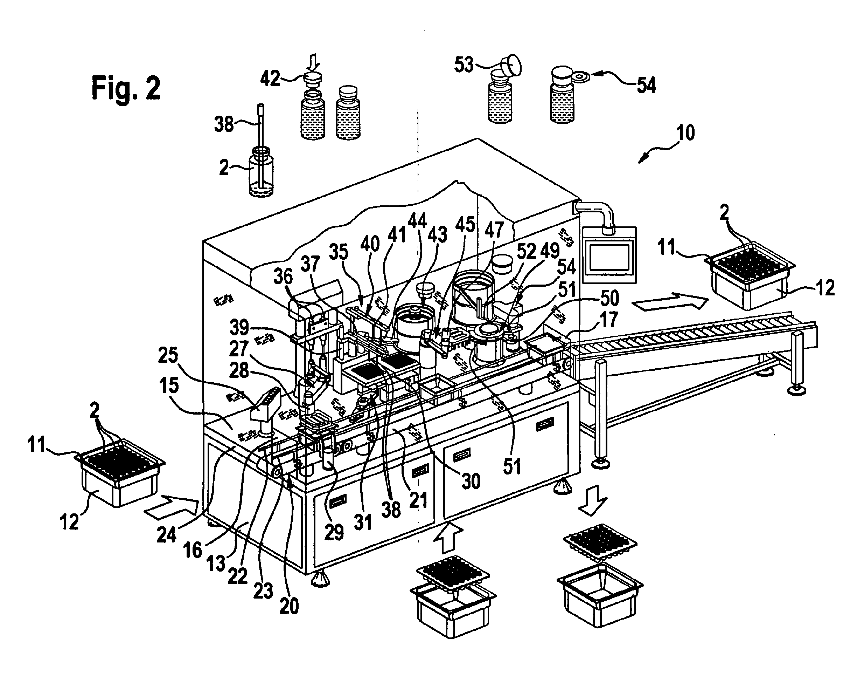

- Fig. 2: die Vorrichtung gemäß der Fig. 1 bei der Verarbeitung von Vials in einer teilweise aufgeschnittenen, perspektivischen Darstellung und

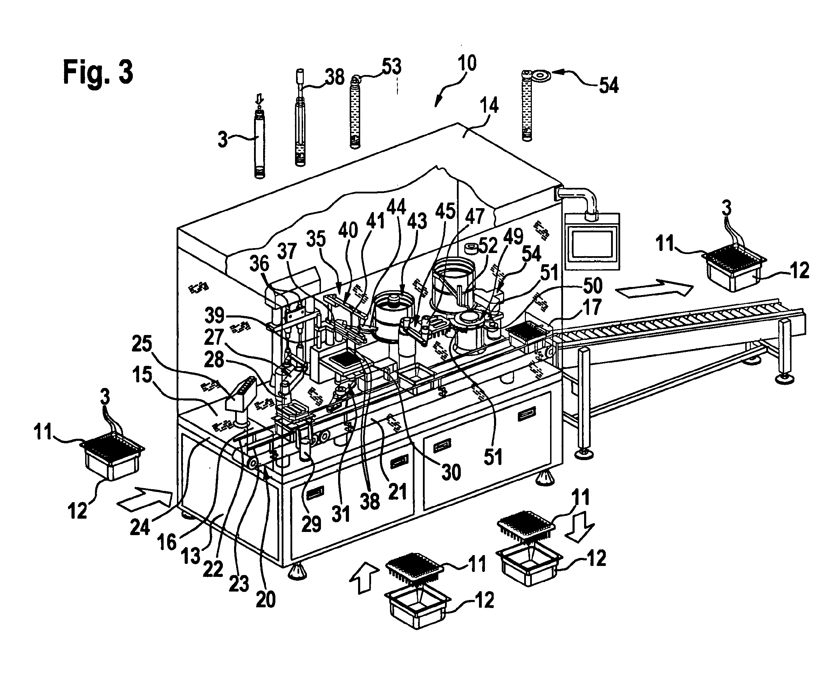

- Fig. 3: die Vorrichtung gemäß der Fig. 1 und 2 bei der Verarbeitung von Zylinderampullen ebenfalls in teilweise aufgeschnittener, perspektivischer Ansicht.

Gleiche Bauteile sind in den Figuren mit denselben Bezugsziffern versehen. Die in den Figuren dargestellte Vorrichtung 10 zum Füllen und Verschließen von pharmazeutischen Behältnissen eignet sich sowohl zum Füllen und Verschließen von Spritzenkörpern 1, als auch von Vials 2 und Zylinderampullen 3. Hierbei werden die Spritzenkörper 1, Vials 2 bzw. Zylinderampullen 3 jeweils in einem mit Aufnahmen versehenen, zumindest im Wesentlichen plattenförmigen Aufnahmeelement 11 der Vorrichtung 10 zugefördert, wobei das Aufnahmeelement 11 in einem wannenförmigen Aufnahmebehälter 12 eingesetzt und dort durch Formschluss positioniert ist. Der Aufnahmebehälter 12 ist mittels einer nicht dargestellten Abdeckung verschließbar, die dem Transportschutz dient. Diese Ausbildung hat den Vorteil, dass die Spritzenkörper 1, Vials 2 bzw. Zylinderampullen 3 beim Hersteller der pharmazeutischen Behältnisse bereits (vor-) sterilisiert werden können, so dass diese beim Pharmazeuten lediglich noch befüllt und verschlossen werden müssen.

Die Vorrichtung 10 weist ein kastenartiges Gehäuse 13 auf, in dessen Innenraum ein von dem Deckenbereich 14 in Richtung des Bodenbereichs 15 des Gehäuses 13 gerichteter Laminarflow (nicht dargestellt) mittels einer Gebläseeinrichtung erzeugbar ist. An der einen Stirnseite des Gehäuses 13 ist eine Einführschleuse 16 für die Aufnahmebehälter 12 angeordnet und an der ihr gegenüberliegenden Stirnseite des Gehäuses 13 befindet sich eine Ausführschleuse 17 für die Aufnahmebehälter 12. Innerhalb des Gehäuses 13 befindet sich an dessen einer Längsseite zwischen der Einführschleuse 16 und der Ausführschleuse 17 eine Fördereinrichtung 20 für die Aufnahmebehälter 12.

Die Fördereinrichtung 20 besteht im Ausführungsbeispiel aus einem Förderband 21 sowie zwei Führungsschienen 22, 23, die die Aufnahmebehälter 12 während ihres Transports durch die Vorrichtung 10 seitlich führen. Die geradlinig ausgebildete Fördereinrichtung 20 befindet sich, wie bereits erläutert, an der einen Seite innerhalb des Gehäuses 13. Auf einer Tischplatte 24 im Gehäuse 13 sind auf der anderen Seite des Gehäuses 13 Handhabungseinrichtungen für die Behältnisse angeordnet. So erkennt man nahe der Einführschleuse 16 eine Wiegeeinrichtung 25, die ein gleichzeitiges Wiegen von mehreren pharmazeutischen Behältnissen ermöglicht. An die Wiegeeinrichtung 25 schließt sich eine Handhabungseinrichtung in Form eines Handhabungsroboters 27 an, dessen Greifarm 28 es in Verbindung mit einer Halteeinrichtung 29 ermöglicht, das Aufnahmeelement 11 mitsamt den darin angeordneten pharmazeutischen Behältnissen aus dem Aufnahmebehälter 12 zu entnehmen und das Aufnahmeelement 11 auf eine Transportplatte 30 abzusetzen.

Die Transportplatte 30 ist Teil einer weiteren Transporteinrichtung 31, die das Aufnahmeelement 11 kontinuierlich oder taktweise bewegt. An den Handhabungsroboter 27 schließt sich eine Füll- und Verschließeinrichtung 35 an. Die Füll- und Verschließeinrichtung 35 umfasst mehrere Förderpumpen 36, mittels der Pharmazeutika masse- bzw. volumengenau dosiert werden können. Die Förderpumpen 36 sind mit einer heb- und senkbaren Füllnadelhalter 37 verbunden. An dem Füllnadelhalter 37 ist beispielhaft für jedes Behältnis eine Füllnadel 38 angeordnet.

Dem Füllnadelhalter 37 ist weiterhin optional wenigstens ein erstes Setzrohr 39 vorgeschaltet, welches in jedes Behältnis eine bestimmte Anzahl von Kugeln einführen kann, um eine bessere Durchmischung der Pharmazeutika vor der Verabreichung zu bewirken.

An den Füllnadelhalter 37 schließt sich eine galgenartige Verschließeinrichtung 40 für Spritzenkörper 1 an. Die Verschließeinrichtung 40 umfasst für jeden Spritzenkörper 1 ein zweites Setzrohr 41, über die ein Verschlussstopfen 42 in den Spritzenkörper 1 eingesetzt werden kann.

Wesentlich ist noch, dass der Handhabungsroboter 27 bzw. der Bewegungsweg seines Greifarms 28 bis in den Bereich der Füll- und Verschließeinrichtung 35 reicht, so dass der Handhabungsroboter 27 mittels seines Greifarms 28 nicht nur unbefüllte Behältnisse aus dem Aufnahmeelement 11 entnehmen kann, sondern auch bereits befüllte Behältnisse, um diese der Wiegeeinrichtung 25 zuzuführen.

Um die Verschlussstopfen 42 zu bevorraten bzw. den zweiten Setzrohren 41 zuzuführen, ist weiterhin ein Verschlussstopfenvorratsbehälter 43 vorgesehen, der über Längsfördereinheiten 44 mit den zweiten Setzrohren 41 gekoppelt ist.

Im Bereich des Verschlussstopfenvorratsbehälters 43 ist weiterhin eine weitere Handhabungseinrichtung in Form eines zweiten Handhabungsroboters 45 angeordnet. Der Handhabungsroboter 45 weist einen Greifarm 47 auf, der es ermöglicht, je nach Art eines an dem Greifarm 47 montierten Formatteils entweder die in dem Aufnahmeelement 11 in Reihen neben- und hintereinander angeordneten, und zuvor in der Füll- und Verschließeinrichtung 35 befüllten Spritzenkörper 1 zu entnehmen und wieder in den Aufnahmebehälter 12 einzusetzen, oder aber, bei der Handhabung von Vials 2 bzw. Zylinderampullen 3, jeweils eine bestimmte Anzahl von Vials 2 bzw. Zylinderampullen 3 an eine nachgeordnete Zufördereinrichtung 49 abzugeben.

Die Zufördereinrichtung 49 weist ein Förderrad 50 mit daran an seinem Umfang beweglich angeordneten Aufnahmesegmenten 51 auf. Mittels der Aufnahmesegmente 51, die sich während der Übernahme von Vials 2 bzw. Zylinderampullen 3 drehen, werden die Vials 2 bzw. Zylinderampullen 3 unter einer Kappenabzugseinrichtung 52 vorbeigeführt; die Teil einer Bördelkappenbevorratungseinrichtung ist, wobei beim Vorbeiführen der Vials 2 bzw. der Zylinderampullen 3 die Vials 2 bzw. die Zylinderampullen 3 jeweils eine Bördelkappe 53 von der Kappenabzugseinrichtung 52 entnehmen bzw. abziehen.

In einer nicht dargestellten Variante ist es jedoch auch denkbar, dass der Greifarm 47 des Handhabungsroboters 45 aus einer Bevorratungsstation für die Bördelkappen 53 eine entsprechende Anzahl von Bördelkappen 53 entnimmt und vor dem Entnehmen der Vials 2 bzw. Zylinderampullen 3 aus dem Aufnahmeelement 11 die Bördelkappen 53 auf die Vials 2 bzw. Zylinderampullen 3 aufsetzt und diese an einander gegenüberliegenden Seiten vorbördelt.

Während der weiteren Bewegung der Aufnahmesegmente 51 am Förderrad 50 gelangen die Vials 2 bzw. Zylinderampullen 3 in den Bereich einer Bördelstation 54, an der die Bördelkappen 53 auf den Vials 2 bzw. Zylinderampullen 3 gebördelt werden. Anschließend wird das Aufnahmesegment 51 wieder an eine Übergabeposition verbracht, die der Übernahmeposition entspricht, in der der Handhabungsroboter 45 die Vials 2 bzw. die Zylinderampullen 3 in die Aufnahmesegmente 51 übergeben hat.

Die soweit beschriebene Vorrichtung 10 arbeitet wie folgt: Beim Verarbeiten von Spritzenkörpern 1 wird das Aufnahmeelement 11 mittels des Handhabungsroboters 27 aus dem Aufnahmebehälter 12 entnommen und auf die Transportplatte 30 abgesetzt. Anschließend kann optional vorgesehen sein, eine bestimmte Anzahl von Spritzenkörpern 1 aus dem Aufnahmeelement 11 zu entnehmen und auf der Wiegeeinrichtung 25 vor dem Füllen zu wiegen. Anschließend werden die Spritzenkörper 1 auf ihrem Förderweg in der Transporteinrichtung 31 mittels der Füll- und Verschließeinrichtung 35 mit einer definierten Füllmenge an Pharmazeutika befüllt. Anschließend kann es vorgesehen sein, die zuvor gewogenen, befüllten Spritzenkörper 1 mittels des Handhabungsroboters 27 nochmals der Wiegeeinrichtung 25 zuzuführen, um zu kontrollieren, ob die richtige Füllmenge eindosiert wurde. Anschließend werden die Verschlusssstopfen 42 in die Spritzenkörper 1 eingebracht und das Aufnahmeelement 11 mittels des zweiten Handhabungsroboters 45 wieder in den Aufnahmebehälter 12 verbracht. Dieser wird anschließend mittels der Fördereinrichtung 20 aus dem Gehäuse 13 bzw. der Vorrichtung 10 ausgeschleust.

Die Verarbeitung von Vials 2 anstelle von Spritzenkörpern 1 unterscheidet sich von der zuvor beschriebenen Handhabung von Spritzenkörpern 1 dadurch, dass der zweite Handhabungsroboter 45 die zuvor befüllten, und ebenfalls mit Verschlussstopfen 42 versehenen Vials 2 nach dem Befüllen und Verschließen mit den Verschlussstopfen 42 reihen- bzw. satzweise aus dem Aufnahmeelement 11 entnimmt und an ein Aufnahmesegment 51 des Förderrades 50 abgibt. Anschließend werden die Vials 2 mittels der Bördelstation 54 gebördelt. Dann entnimmt der zweite Handhabungsroboter 45 die gebördelten Vials 2 wieder dem Aufnahmesegment 51 in seiner ursprünglichen Position und setzt die Vials 2 in die entsprechenden Aufnahmen des Aufnahmeelementes 11 ein. Sobald alle Vials 2 aus einem Aufnahmeelement 11 vollständig gebördelt sind, wird das Aufnahmeelement 11 mittels des Handhabungsroboters 45 wieder in den Aufnahmebehälter 12 eingesetzt und anschließend mittels der Fördereinrichtung 20 aus der Vorrichtung 10 ausgeschleust.

Die Verarbeitung von Zylinderampullen 3 anstelle von Vials 2 unterscheidet sich von der zuvor beschriebenen Behandlung der Vials 2 insbesondere dadurch, dass bei den Zylinderampullen 3 vorher keine Verschlussstopfen gesetzt werden. Insofern ist es besonders wichtig, dass der Laminarflow in der Vorrichtung 10 nach dem Befüllen der Zylinderampullen 3 möglichst wenig gestört wird bzw. ein möglichst kurzer Förderweg der Zylinderampullen 3 mit Bördelkappen 53 vorgesehen ist, um die Gefahr des Herabfallens der Bördelkappen 53 zu minimieren, so dass entweder die Bördelkappen 53 an der Kappenabzugseinrichtung 52 erst unmittelbar vor dem Bördeln abzogen werden, oder stattdessen der zweite Handhabungsroboter 45 bei der Entnahme der Zylinderampullen 3 aus dem Aufnahmeelement 11 die Zylinderampullen 3, wie vorab beschrieben, bereits mit den Bördelkappen 53 versieht.

Die soweit beschriebene Vorrichtung 10 kann in vielfältiger Art und Weise abgewandelt bzw. modifiziert werden. So ist es beispielsweise denkbar, die Vials 2 bzw. Zylinderampullen 3 direkt nach dem Befüllen mittels des zweiten Handhabungsroboters 45 zu entnehmen und dem Aufnahmesegment 51 zuzufördem. Auch ist es denkbar, nach dem Befüllen das Aufnahmeelement 11 wieder in den Aufnahmebehälter 12 einzusetzen und das Aufnahmeelement 11 erst im Bereich einer Bördeleinrichtung wieder aus dem Aufnahmebehälter 12 zu entnehmen. Weiterhin kann die Übergabe der Behältnisse von dem zweiten Handhabungsroboter an die Aufnahmesegmente 51 und umgekehrt auch in einer stillstandsphase der Aufnahmesegmente 51 erfolgen.

## Patentansprüche

1. Vorrichtung (10) zum Füllen und Verschließen von pharmazeutischen Behältnissen, wobei die Behältnisse in einem insbesondere wannenförmigen Aufnahmebehälter (12) aufgenommen sind, in dem ein aus dem Aufnahmebehälter (12) entnehmbares Trägerelement (11) eingesetzt ist, in dem die Behältnisse in mehreren Reihen neben- und hintereinander in Aufnahmen des Trägerelements (11) angeordnet sind, mit einer ersten Handhabungseinheit (27) zum Entnehmen des Trägerelements (11) aus dem Aufnahmebehälter (12), einer Füll- und Verschließeinrichtung (35) für die Behältnisse und einer zweiten Handhabungseinheit (45) zum Wiedereinsetzen des Trägerelements (11) in den auf einer Fördereinrichtung (20) mit dem Trägerelement (11) mitgeförderten Aufnahmebehälter (12),
**dadurch gekennzeichnet,**
**dass** die Behältnisse als Spritzenkörper (1) oder als mit Bördelkappen (53) zu versehende Behältnisse wie Vials (2) oder Zylinderampullen (3) ausgebildet sind und, dass der zweiten Handhabungseinrichtung (45) eine Bördeleinrichtung (54) zugeordnet ist, die beim Handhaben von mit Bördelkappen (53) zu versehenden Behältnissen von der zweiten Handhabungseinrichtung (45) angesteuert wird, so dass die zweite Handhabungseinrichtung (45) die Behältnisse zunächst der Bördeleinrichtung (54) zuführt und anschließend die zuvor mittels der Bördeleinrichtung (54) verschlossenen Behältnisse wieder in das Trägerelement (11) einsetzt.

2. Vorrichtung nach Anspruch 1,
**dadurch gekennzeichnet,**
**dass** der Bördeleinrichtung (54) eine Zufördereinrichtung (49) mit Aufnahmeelementen (51) für die Behältnisse vorgeschaltet ist und, dass die Aufnahmeelemente (51) der Zufördereinrichtung (49) einen Übernahmebereich für die Behältnisse aus dem Trägerelement (11) und einen Übergabebereich der Behältnisse an das Trägerelement (11) aufweist, in der die Aufnahmeelemente (51) während der Übergabe bzw. Übernahme der Behältnisse mittels der zweiten Handhabungseinheit (45) zusammen mit der zweiten Handhabungseinheit (45) aneinander vorbeibewegt werden.

3. Vorrichtung nach Anspruch 1 oder 2,
**dadurch gekennzeichnet,**
**dass** zumindest die zweite Handhabungseinheit als Handhabungsroboter (45) ausgebildet ist, dessen mit den Behältnissen in Wirkverbindung angeordneter Greifarm (47) bezüglich seines Bewegungswegs horizontal und vertikal frei programmierbar ist.

4. Vorrichtung nach Anspruch einem der Ansprüche 1 bis 3,
**dadurch gekennzeichnet,**
**dass** der Bördeleinrichtung (54) unmittelbar eine Bördelkappenübergabeeinrichtung (52) vorgeschaltet ist, in der die Behältnisse jeweils eine Bördelkappe (53) übernehmen.

5. Vorrichtung nach Anspruch 3,
**dadurch gekennzeichnet,**
**dass** der Greifarm (47) vor der Entnahme der Behältnisse aus dem Träger element (11) an einer Bördelkappenbevorratungseinrichtung vorbeigeführt wird, in der der Greifarm (47) Bördelkappen (53) entnimmt und, dass der Greifarm (47) vor der Entnahme der Behältnisse aus dem Trägerelement (11) auf den Kopfbereich jedes Behältnisses eine Bördelkappe (53) aufsetzt.

6. Vorrichtung nach Anspruch 5,
**dadurch gekennzeichnet,**
**dass** der Greifarm (47) bei Aufsetzen der Bördelkappe (53) auf das Behältnis die Bördelkappe (53) in Wirkverbindung mit dem Behältnis bringt, so dass die Bördelkappe (53) auf dem Behältnis vorgebördelt ist.

7. Vorrichtung nach einem der Ansprüche 1 bis 6,
**dadurch gekennzeichnet,**
**dass** die Vorrichtung (10) in einem, mit Ausnahme einer Einführschleuse (16) und einer Ausführschleuse (17), geschlossenen Gehäuse (13) aufgenommen ist und, dass eine Gebläseeinrichtung vorgesehen ist, die innerhalb des Gehäuses (13) einen vom Deckenbereich (14) in Richtung des Bodenbereichs (15) des Gehäuses (13) gerichteten Laminarflow erzeugt.

8. Vorrichtung nach einem der Ansprüche 1 bis 7,
**dadurch gekennzeichnet,**
**dass** zumindest die Füll- und Verschließeinrichtung (35) bzw. die Bördeleinrichtung (54) und die der Bördeleinrichtung (54) zugeordneten Einrichtungen bzw. eine Transportplatte (30) bzw. Greifarme (28, 47) der Handhabungseinheiten (27, 45) an standardisierten Aufnahmeelementen austauschbar befestigt sind.

9. Vorrichtung nach Anspruch 7 oder 8,
**dadurch gekennzeichnet,**
**dass** die Fördereinrichtung (20) für den Aufnahmebehälter (12) geradlinig ausgebildet ist und sich zwischen der Einführschleuse (16) und der Ausführschleuse (17) im Gehäuse (13) erstreckt und, dass zumindest die Handhabungseinheiten (27, 45), die Füll- und Verschließeinrichtung (35) und die Bördeleinrichtung (54) sowie die der Bördeleinrichtung zugeordneten Einrichtungen seitlich neben der Fördereinrichtung (20) auf einer gemeinsamen Seite innerhalb des Gehäuses (13) angeordnet sind.

10. Vorrichtung nach einem der Ansprüche 1 bis 9,
**dadurch gekennzeichnet,**
**dass** die erste Handhabungseinheit als Handhabungsroboter (27) ausgebildet ist, dessen mit den Behältnissen in Wirkverbindung angeordneter Greifarm (28) bezüglich seines Bewegungswegs horizontal und vertikal frei programmierbar ist und, dass im Bewegungsweg des Greifarms (28) wenigstens eine Wiegeeinrichtung (25) für die Behältnisse angeordnet ist.

## Claims

1. Device (10) for filling and sealing pharmaceutical containers, wherein the containers are accommodated in an, in particular, tub-shaped receptacle (12), in which is inserted a carrier element (11) which is removable from the receptacle (12) and in which the containers are arranged side by side and one behind the other in multiple rows in receiving fixtures of the carrier element (11), said device comprising a first handling unit (27) for removing the carrier element (11) from the receptacle (12), a filling and sealing device (35) for the containers, and a second handling unit (45) for reinserting the carrier element (11) into the receptacle (12), which latter is transported along with the carrier element (11) on a conveyor (20),
**characterized**
**in that** the containers are configured as syringe barrels (1) or as containers, such as vials (2) or cartridges (3), to be provided with crimp caps (53), and in that to the second handling device (45) there is assigned a crimping device (54), which, in the handling of containers to be provided with crimp caps (53), is controlled by the second handling device (45), so that the second handling device (45) first feeds the containers to the crimping device (54) and then inserts the containers which have previously been sealed by means of the crimping device (54) back into the carrier element (11).

2. Device according to Claim 1,
**characterized**
**in that** a feed device (49) having receiving elements (51) for the containers is placed upstream of the crimping device (54), and in that the receiving elements (51) of the feed device (49) have a take-up region for the containers from the carrier element (11) and a delivery region for the containers to the carrier element (11), in which the receiving elements (51), during the delivery or take-up of the containers by means of the second handling unit (45), are moved past one another together with the second handling unit (45).

3. Device according to Claim 1 or 2,
**characterized**
**in that** at least the second handling unit is configured as a handling robot (45), whose gripper arm (47) arranged in operative connection with the containers is freely programmable in the horizontal and vertical directions with respect to its motional path.

4. Device according to one of Claims 1 to 3,
**characterized**
**in that** a crimp cap delivery device (52), in which the containers respectively take up a crimp cap (53), is placed directly upstream of the crimping device (54).

5. Device according to Claim 3,
**characterized**
**in that** the gripper arm (47), prior to the removal of the containers from the carrier element (11), is guided past a crimp cap supplying device, in which the gripper arm (47) removes crimp caps (53), and in that the gripper arm (47), prior to the removal of the containers from the carrier element (11), places a crimp cap (53) onto the head region of each container.

6. Device according to Claim 5,
**characterized**
**in that** the gripper arm (47), when the crimp cap (53) is placed onto the container, brings the crimp cap (53) into operative connection with the container, so that the crimp cap (53) is precrimped onto the container.

7. Device according to one of Claims 1 to 6,
**characterized**
**in that** the device (10) is accommodated in a, with the exception of a feed-in gate (16) and a feed-out gate (17), closed housing (13), and in that a blower device is provided, which generates within the housing (13) a laminar flow directed from the ceiling region (14) in the direction of the floor region (15) of the housing (13).

8. Device according to one of Claims 1 to 7,
**characterized**
**in that** at least the filling and sealing device (35), or the crimping device (54) and the devices assigned to the crimping device (54), or a transport plate (30) or gripper arms (28, 47) of the handling units (27, 45), are exchangeably fastened to standardized receiving elements.

9. Device according to Claim 7 or 8,
**characterized**
**in that** the conveyor (20) for the receiving container (12) is of rectilinear configuration and extends between the feed-in gate (16) and the feed-out gate (17) in the housing (13), and in that at least the handling units (27, 45), the filling and sealing device (35) and the crimping device (54), as well as the devices assigned to the crimping device, are disposed alongside the conveyor (20) on a common side within the housing (13).

10. Device according to one of Claims 1 to 9,
**characterized**
**in that** the first handling device is configured as a handling robot (27), whose gripper arm (28) arranged in operative connection with the containers is freely programmable in the horizontal and vertical directions with respect to its motional path, and in that, in the motional path of the gripper arm (28), at least one weighing device (25) for the containers is arranged.

## Revendications

1. Dispositif (10) pour remplir et fermer des contenants pharmaceutiques, les contenants étant reçus dans un récipient de réception (12) en particulier en forme de bac, dans lequel est introduit un élément de support (11) pouvant être retiré hors du récipient de réception (12), dans lequel les contenants sont disposés en plusieurs rangées les uns à côté des autres et les uns derrière les autres dans des logements de l'élément de support (11), comprenant une première unité de manipulation (27) pour retirer l'élément de support (11) hors du récipient de réception (12), un dispositif de remplissage et de fermeture (35) pour les contenants et une deuxième unité de manipulation (45) pour réintroduire l'élément de support (11) dans le récipient de réception (12) transporté conjointement avec l'élément de support (11) sur un dispositif de transport (20),
**caractérisé en ce que**
les contenants sont réalisés sous forme de corps de seringue (1) ou sous forme de contenants à pourvoir de capuchons sertis (53), comme des flacons (2) ou des ampoules cylindriques (3), et **en ce qu'**un dispositif de sertissage (54) est associé au deuxième dispositif de manipulation (45), lequel dispositif de sertissage est commandé par le deuxième dispositif de manipulation (45) lors de la manipulation de contenants à pourvoir de capuchons sertis (53), de sorte que le deuxième dispositif de manipulation (45) achemine tout d'abord les contenants jusqu'au dispositif de sertissage (54) et ensuite introduise à nouveau dans l'élément de support (11) les contenants fermés préalablement au moyen du dispositif de sertissage (54).

2. Dispositif selon la revendication 1,
**caractérisé en ce**
**qu'**un dispositif de transport (49) doté d'éléments de réception (51) pour les contenants est placé avant le dispositif de sertissage (54) et en ce que les éléments de réception (51) du dispositif de transport (49) comprennent une région de prise pour les contenants à partir de l'élément de support (11) et une région de transfert des contenants à l'élément de support (11), les éléments de réception (51) étant déplacés les uns devant les autres conjointement avec la deuxième unité de manipulation (45) pendant la prise et le transfert des contenants au moyen de la deuxième unité de manipulation (45).

3. Dispositif selon la revendication 1 ou 2,
**caractérisé en ce**
**qu'**au moins la deuxième unité de manipulation est réalisée sous forme de robot de manipulation (45) dont le bras de préhension (47) disposé en liaison fonctionnelle avec les contenants peut être programmé librement horizontalement et verticalement en ce qui concerne sa course de déplacement.

4. Dispositif selon l'une quelconque des revendications 1 à 3,
**caractérisé en ce**
**qu'**un dispositif de transfert de capuchon serti (52) est placé directement avant le dispositif de sertissage (54), dans lequel dispositif de transfert les contenants reçoivent respectivement un capuchon serti (53).

5. Dispositif selon la revendication 3,
**caractérisé en ce**
**que** le bras de préhension (47) est guidé devant un dispositif de stockage de capuchons sertis avant le retrait des contenants hors de l'élément de support (11), dans lequel dispositif de stockage le bras de préhension (47) prélève des capuchons sertis (53), et en ce que le bras de préhension (47) place sur la région de tête de chaque contenant un capuchon serti (53) avant le retrait des contenants hors de l'élément de support (11).

6. Dispositif selon la revendication 5,
**caractérisé en ce**
**que** le bras de préhension (47) amène le capuchon serti (53) en liaison fonctionnelle avec le contenant lors du placement du capuchon serti (53) sur le contenant, de sorte que le capuchon serti (53) soit pré-serti sur le contenant.

7. Dispositif selon l'une quelconque des revendications 1 à 6,
**caractérisé en ce**
**que** le dispositif (10) est reçu dans un boîtier (13) fermé par réception d'un sas d'introduction (16) et d'un sas de sortie (17), et en ce qu'il est prévu un dispositif de soufflerie qui produit à l'intérieur du boîtier (13) un écoulement laminaire orienté à partir de la région de recouvrement (14) en direction de la région de fond (15) du boîtier (13).

8. Dispositif selon l'une quelconque des revendications 1 à 7,
**caractérisé en ce**
**qu'**au moins le dispositif de remplissage et de fermeture (35), respectivement le dispositif de sertissage (54) et les dispositifs associés au dispositif de sertissage (54), respectivement une plaque de transport (30), respectivement des bras de préhension (28, 47) des unités de manipulation (27, 45) sont fixés de manière remplaçable à des éléments de réception standardisés.

9. Dispositif selon la revendication 7 ou 8,
**caractérisé en ce**
**que** le dispositif de transport (20) pour le récipient de réception (12) est rectiligne et s'étend entre le sas d'introduction (16) et le sas de sortie (17) dans le boîtier (13), en ce qu'au moins les unités de manipulation (27, 45), le dispositif de remplissage et de fermeture (35) et le dispositif de sertissage (54) ainsi que les dispositifs associés au dispositif de sertissage sont disposés latéralement à côté du dispositif de transport (20) d'un côté commun à l'intérieur du boîtier (13).

10. Dispositif selon l'une quelconque des revendications 1 à 9,
**caractérisé en ce**
**que** la première unité de manipulation est réalisée sous forme de robot de manipulation (27) dont le bras de préhension (28) disposé en liaison fonctionnelle avec les contenants peut être programmé librement horizontalement et verticalement en ce qui concerne sa course de déplacement et en ce qu'au moins un dispositif de pesage (25) pour les contenants est disposé sur la course de déplacement du bras de préhension (28).
